(19) Europäisches Patentamt
European Patent Office
Office européen des brevets

(11) EP 1 234 835 A2

# (12) EUROPEAN PATENT APPLICATION

(43) Date of publication:
**28.08.2002 Bulletin 2002/35**

(51) Int Cl.[7]: **C07K 14/47**, G01N 33/68, G01N 33/542, C12Q 1/48

(21) Application number: **02251083.8**

(22) Date of filing: **18.02.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **23.02.2001 US 271102 P**

(71) Applicant: **PHARMACIA & UPJOHN COMPANY**
**Kalamazoo, Michigan 49001 (US)**

(72) Inventors:
- **Rank, Kenneth B.**
  **Mattawan, Michigan 49071 (US)**
- **Sharma, Satish K.**
  **Protage, Michigan 49024 (US)**

(74) Representative: **Perry, Robert Edward**
**GILL JENNINGS & EVERY**
**Broadgate House**
**7 Eldon Street**
**London EC2M 7LH (GB)**

## (54) Assays for assessing ABeta-Tau Aggregation

(57) The present invention deals with the field of cell biology, more specifically the field of neuronal cell biology and neurodegenerative diseases. The invention provides for assays assessing Aβ-Tau aggregation

EP 1 234 835 A2

## Description

### CROSS REFERENCE TO RELATED APPLICATIONS

The present application claims priority of Application Serial Number 60/271102 filed 23 February 2001 which is hereby incorporated by reference.

### Field of the Invention

The present invention deals with the field of cell biology, more specifically the field of neuronal cell biology and neurodegenerative diseases. The invention provides for assays assessing Aβ-Tau aggregation

### Background

In Alzheimer's disease, Aβ peptide and hyperphosphorylated tau are found in the amyloid plaques and neurofibrillary tangles, respectively. Recently, it has been suggested that Aβ peptide oligomerization begins intraneuronally and the earliest detectable dementia in AD arises from the onset of tau aggregation. Six isoforms of human tau are expressed in adult human brain. (1) These arise from alternate splicing of the mRNA transcribed from a single gene located on the long arm of chromosome 17 (1-5) One of the functions of the tau protein is to promote microtubule assembly *in vivo* and to stabilize microtubules in the nervous system (6-10).

Neurofibrillary tangles (NFT) and senile plaques constitute two prominent neuropathological characteristics of Alzheimer's disease (11,12). The main fibrous component of all neurofibrillary lesions is paired helical filament (PHF) which contains predominantly the abnormally phosphorylated tau (13-18). It has been hypothesized that aberrant phosphorylation of tau leads to its aggregation into PHF, resulting in destabilization of microtubules and the death of neurons (1,19). Notably, hyperphosphorylated tau has been detected in other tau-positive filamentous lesions, in a group of diseases collectively known as the tauopathies. These neurodegenerative diseases include Progressive Supranuclear Palsy (PSP), Corticobasal Degeneration (CBD), Down's syndrome, Frontotemporal dementia and Parkinsonism linked to chromosome 17 (FTDP), and Pick's disease (20). Recent studies show that these various phenotypes might be the result of phosphorylation of specific tau isoforms in different nerve cells in distinct brain regions (21).

The upstream factors and small molecule modulators of tau aggregation have remained elusive however. Assays to access this phenomenon are a valuable tool in the discovery of agents potentially useful in the treatment and prevention of a variety of neurodegenerative diseases.

### References Cited

1. Goedert, M., *et al., Multiple isoforms of human microtubule-associated protein tau: sequences and localization in neurofibrillary tangles of Alzheimer's disease.* Neuron, 1989. **3**(4): p. 519-26.
2. Lee, G., N. Cowan, and M. Kirschner, *The primary structure and heterogeneity of tau protein from mouse brain.* Science, 1988. **239**(4837): p.
285-8.
3. Goedert, M., *et al., Cloning and sequencing of the cDNA encoding a core protein of the paired helical filament of Alzheimer disease: identification as the microtubule-associated protein tau.* Proc Natl Acad Sci U S A, 1988. **85** (11): p. 4051-5.
4. Himmler, A., *Structure of the bovine tau gene: alternatively spliced transcripts generate a protein family.* Mol Cell Biol, 1989. 9(4): p. 1389-96.
5. Kosik, K.S., C.L. Joachim, and D.J. Selkoe, *Microtubule-associated protein tau (tau) is a major antigenic component of paired helical filaments in Alzheimer disease.* Proc Natl Acad Sci U S A, 1986. **83**(11): p. 4044-8.
6. Caceres, A. and K.S. Kosik, *Inhibition of neurite polarity by tau antisense oligonucleotides in primary cerebellar neurons.* Nature, 1990. **343**(6257): p. 461-3.
7. Drubin, D.G. and M.W. Kirschner, *Tau protein function in living cells.* J Cell Biol, 1986. **103**(6 Pt 2): p. 2739-46.
8. Goedert, M. and R. Jakes, *Expression of separate isoforms of human tau protein: correlation with the tau pattern in brain and effects on tubulin polymerization.* Embo J, 1990. **9**(13): p. 4225-30.
9. Horio, T. and H. Hotani, *Visualization of the dynamic instability of individual microtubules by dark-field microscopy.* Nature, 1986. **321**(6070): p. 605-7.
10. Weingarten, M.D., *et al., A protein factor essential for microtubule assembly.* Proc Natl Acad Sci U S A, 1975. 72(5): p. 1858-62.

11. Kosik, K.S., *Alzheimer plaques and tangles: advances on both fronts.* Trends Neurosci, 1991. **14**(6): p. 218-9.
12. Lee, V.M. and J.Q. Trojanowski, *The disordered neuronal cytoskeleton in Alzheimer's disease.* Curr Opin Neurobiol, 1992. **2**(5): p. 653-6.
13. Bancher, C., *et al., Accumulation of abnormally phosphorylated tau precedes the formation of neurofibrillary tangles in Alzheimer's disease.* Brain Res, 1989. **477**(1-2): p. 90-9.
14. Bondareff, W., *et al*., *Molecular analysis of neurofibrillary degeneration in Alzheimer's disease. An immunohistochemical study.* Am J Pathol, 1990. **137**(3): p. 711-23.
15. Goedert, M., *Tau protein and the neurofibrillary pathology of Alzheimer's disease.* Trends Neurosci, 1993. **16**(11): p. 460-5.
16. Greenberg, S.G. and P. Davies, *A preparation of Alzheimer paired helical filaments that displays distinct tau proteins by polyacrylamide gel electrophoresis.* Proc Natl Acad Sci U S A, 1990. **87**(15): p. 5827-31.
17. Ksiezak-Reding, H. and S.H. Yen, *Structural stability of paired helical filaments requires microtubule- binding domains of tau: a model for self-association.* Neuron, 1991. **6**(5): p. 717-28.
18. Lee, V.M., *et al*., *A68: a major subunit of paired helical filaments and derivatized forms of normal Tau.* Science, 1991. **251**(4994): p. 675-8.
19. Kosik, K.S., *Tau protein and Alzheimer's disease.* Curr Opin Cell Biol, 1990. **2**(1): p. 101-4.
20. Spillantini, M.G. and M. Goedert, *Tau protein pathology in neurodegenerative diseases.* Trends Neurosci, 1998. **21**(10): p. 428-33.
21. Mailliot, C., *et al., Phosphorylation of specific sets of tau isoforms reflects different neurofibrillary degeneration processes.* FEBS Lett, 1998. **433**(3): p. 201-4.
22 Glenner, et al., *Alzheimer's Disease: Initial Report of the Purification and Characterization of a Novel Cerebrovascular Amyloid Protein,* Biochem. Biophys. Res. Commun., 1984, 120:885-890.
23. Evans, D.B., *et al., Tau phosphorylation at serine 396 and serine 404 by human recombinant tau protein kinase II inhibits tau's ability to promote microtubule assembly. J* Biol Chem, 2000. **275**(32): p. 24977-83.

**Brief Description of the Sequence Listing**

SEQ ID NO: 1 Tau 441 cDNA nucleotide sequence
SEQ ID NO:2 Tau 441 protein sequence
SEQ ID NO:3 Tau 383 cDNA nucleotide sequence
SEQ ID NO:4 Tau 383 protein sequence
SEQ ID NO:5 Tau 352 cDNA nucleotide sequence
SEQ ID NO:6 Tau 352 protein sequence
SEQ ID NO:7 Aβ 1-43 peptide sequence

**Brief Description of the Figures**

Figure 1. SPA studying the aggregation of tau in the presence of Aβ 1-42. Tau was [$^{33}$P] labeled using TPK II and then varying concentrations mixed with 2 μM Aβ 1-42 and SPA beads. The mixture was centrifuged and processed as described. CPM was plotted versus [$^{33}$P] tau concentration and the data fitted using a ligand binding 1 site equation.

Figure 2. Time course of the aggregation of tau in the presence of Aβ 1-42. Tau (2 μM) was incubated with 60 μM Aβ 1-42 for the indicated time and the samples centrifuged. The resultant supernatant (S) and pellet (P) were run on SDS-PAGE and Coomassie stained. Lanes 1 and 2, tau alone (no Aβ 1-42) incubated for 2 hours; lanes 3 and 4, tau and Aβ 1-42 after immediate mixing; lanes 5 and 6, tau and Aβ 1-42 incubated for 15 min; lanes 7 and 8, incubated for 30 min; lanes 9 and 10, incubated for 1 hour; lanes 11 and 12, incubated for 2 hours; lane 13, MW markers.

Figure 3. Dose-response of the aggregation of tau in the presence of varying Aβ 1-42. Tau (2 μM) or TPK II phosphorylated tau (2 μM) was incubated with varying Aβ 1-42 for 30 min at 37°C. The samples were processed as described and run on SDS-PAGE. The gel was scanned by densitometry and the percentage of tau in the pellet was calculated and plotted versus Aβ 1-42 concentration.

Figure 4. TPK II phosphorylation of soluble tau and Aβ 1-42 aggregated tau. Tau (10 μM) was incubated in the presence of 60 μM Aβ 1-42 for 30 min at 37°C. The sample was centrifuged and the supernatant (soluble tau) removed from the pellet (aggregated tau). These two samples (2 μM each) were incubated with 3.6 nM TPK II and [$^{33}$P] incorporation followed using the filter-binding assay. Data represents the average of three experiments.

Figure 5. Measurement of Aβ:tau interaction by absorbance measurements

Figure 6. Phosphorylation of tau by TPK II in the presence of Aβ peptides using the filter binding assay. Tau (2 μM) was incubated with 3.6 nM TPK II and varying concentrations of either Aβ 1-42, Aβ 1-40 or Aβ 25-35 as described. The tau phosphorylation was determined by [$^{33}$P] incorporation and the percent tau phosphorylation relative to TPK II phosphorylated tau in the absence of Aβ peptides was plotted versus peptide concentration. Data represents the average of three experiments.

Figure 7. Time course of the phosphorylation of tau by TPK II in the presence of Aβ 1-42. Tau (2 μM) was incubated with or without 60 μM Aβ 1-42 and 3.6 nM TPK II for various times. Phosphorylation was followed by [$^{33}$P] incorporation using the filter-binding assay and plotted as CPM incorporated versus time. Data represents the average of three experiments.

Figure 8. Phosphorylation of tau by TPK II in the presence of Aβ peptides using the gel-shift assay. Tau (3 μM) was incubated with 50 nM TPK II in the presence and absence of 60 μM Aβ peptides for 4 hours and the products run on 12% SDS-PAGE as described. Lane 1, unphosphorylated tau; lane 2, TPK II phosphorylated tau; lane 3, TPK II phosphorylated tau with Aβ 25-35; lane 4, TPK II phosphorylated tau with non-aggregated Aβ 1-40; lane 5, TPK II phosphorylated tau with Aβ 1-42; lane 6, TPK II phosphorylated tau; lane 7, unphosphorylated tau; lane 8, MW markers.

Figure 9. Phosphorylation of tau by TPK II in the presence of Aβ 1-42 in the gel-shift assay. Tau (3 μM) was incubated with 50 nM TPK II in the presence of varying Aβ 1-42 for 4 hours and the products run on 12% SDS-PAGE as described. Lane 1, unphosphorylated tau; lane 2, tau incubated with 60 μM Aβ 1-42; lane 3, TPK II phosphorylated tau; lanes 4-7, TPK II phosphorylated tau with 8 μm, 16 μM, 30 μM, and 60 μM Aβ 1-42 respectively; lane 8, TPK II phosphorylated tau; lane 9, unphosphorylated tau; lane 10, MW markers.

Figure 10. Time course of the phosphorylation of tau by TPK II in the presence of Aβ 1-42 using the gel-shift assay. Tau (3μM) was incubated with 50 nM TPK II in the presence of 60 μM Aβ 1-42 for various times and the products run on 12% SDS-PAGE as described. Lane 1, unphosphorylated tau; lane 2, TPK II phosphorylated tau for 15 min; lane 3, TPK II phosphorylated tau with Aβ 1-42 for 15 min; lane 4, TPK II phosphorylated tau for 30 min; lane 5, TPK II phosphorylated tau with Aβ 1-42 for 30 min; lane 6, TPK II phosphorylated tau for 1 hour; lane 7, TPK II phosphorylated tau with Aβ 1-42 for 1 hour; lane 8, unphosphorylated tau; lane 9, MW markers.

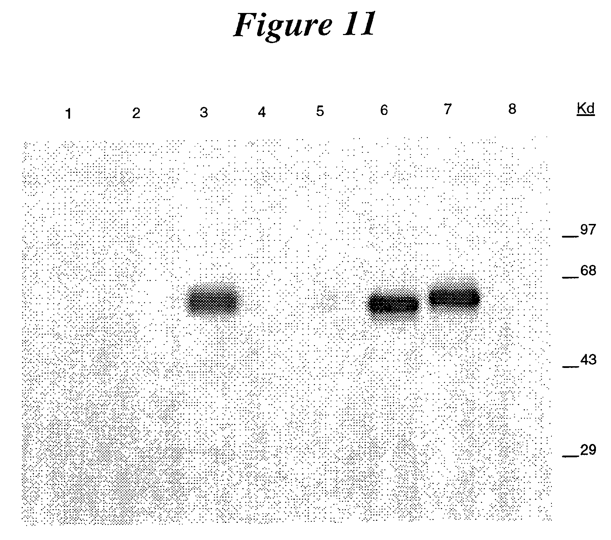

Figure 11. Western blot showing that Aβ 1-42 stimulates phosphorylation of Thr-231 by TPK II. Tau (3 μM) was incubated with TPK I or TPK II in the presence and absence of 60 μM Aβ 1-42 and the samples processed as described for Western blot using the AT180 antibody. Lane 1, unphosphorylated tau; lane 2, TPK II phosphorylated tau; lane 3, TPK II phosphorylated tau with Aβ 1-42; lane 4, TPK I phosphorylated tau; lane 5, TPK I phosphorylated tau with Aβ 1-42; lane 6, TPK II and TPK I phosphorylated tau; lane 7, TPK II and TPK I phosphorylated tau with Aβ 1-42; lane 8, unphosphorylated tau.

## Summary of the Invention

The invention comprises a method for identifying agents that are inhibitors of tau-beta amyloid complex formation comprising contacting a tau protein derived polypeptide and an aggregated beta-amyloid in the presence and absence of a test agent; and determining the amount of tau-beta amyloid complex formed in the presence and absence of the test agent; and comparing the amount of tau-beta amyloid complex formed in the presence of the test agent with the amount of tau-beta amyloid complex formed in the absence of the test agent.

## Detailed Description of the Invention

As used hereinafter "polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Glycosylated and non-glycosylated form of polypeptides are embraced by this definition.

"Synthesized" as used herein and understood in the art, refers to polynucleotides produced by purely chemical, as opposed to enzymatic, methods. "Wholly" synthesized DNA or polypeptide sequences are therefore produced entirely by chemical means, and "partially" synthesized DNAs or polypeptides embrace those wherein only portions of the resulting DNA or polypeptide were produced by chemical means.

"Isolated" as used herein and as understood in the art, whether referring to "isolated" polynucleotides or polypeptides, is taken to mean separated from the original cellular environment in which the polypeptide or nucleic acid is normally found. As used herein therefore, by way of example only, a protein expressed by recombinant means in a cell type in which it does not naturally occur is "isolated". By way of example, a protein species, whether expressed in a naturally occurring cell or not, when purified to any extent is "isolated"

As used herein, the term "contacting" means bringing together, either directly or indirectly, a compound into physical proximity to a polypeptide or of the invention. Additionally "contacting" may mean bringing a polypeptide of the invention into physical proximity with another polypeptide

"Tau protein derived polypeptide" is a polypeptide derived from a mammalian tau comprising the microtubule binding domain of said mamamlian tau or a sequence which is homologous thereto. The microtubule binding domain is characterized by a carboxy-terminal region of three or four imperfect tandem repeats of 31 or 31 amino acids each displaying a Pro-Gly-Gly Gly motif. The term "tau protein derived polypeptide" therefore encompasses any full length mammalian tau isoform as well as fragments thereof. The structural relationship between all the human tau isoforms is elucidated by the table derived from Goedert et al. (1)

| Human Tau Protein Isoforms | | | | |
|---|---|---|---|---|
| Isoform | Inserts | | | Length |
| | 1 2 3 | | | |
| 1 | - | - | - | 352 |
| 2 | - | - | + | 383 |
| 3 | + | - | - | 381 |
| 4 | + | - | + | 412 |
| 5 | + | + | - | 410 |
| 6 | + | + | + | 441 |

The table shows the inserts contained in each isoform. SEQ ID NO: 1 comprises the cDNA sequence of the longest tau isoform. Insert 1 consists of nucleotides 370-456, Insert 2 consists of nucleotides 457-543, and Insert 3 consists of nucleotides 1059-1151. SEQ ID NO:2 comprises the amino acid sequence of the longest tau isoform. Insert 1 therefore consists of amino acid 45-73, Insert 2 therefore consists of amino acid 75-102, and Insert 3 therefore consists of amino acid 275-305.

It should be clear from the foregoing that given the sequence (either DNA or protein) of the longest isoform it is possible to deduce the approximate sequence of any currently known tau isoform. By way of example, a cDNA sequence encoding tau 441 and the protein encoded is represented by SEQ ID NO: 1 and 2 respectively. A cDNA sequence encoding tau 383 and the protein encoded is represented by SEQ ID NO:3 and 4 respectively. A cDNA sequence encoding tau 352 and the protein encoded is represented by SEQ ID NO:5 and 6 respectively.

The microtubule binding domain spans the area between Gln 244- Ala 390 of SEQ ID NO:2 and residues Gln 186- Ala 332 of SEQ ID NO:4 and residues Gln 186-Asn 279 of SEQ ID NO:6. The SEQ ID NOS: representing human tau isoform cDNAs and encoded proteins are included by way of example. Sequences not included are easily obtainable

For simplicity, all numbering in this patent application (unless referring to a specific SEQ ID NO.) refers to the human tau variant containing all exons (441 amino acids long) according to Goedert et al (1) and Genbank # NM_005910

The invention is meant to be practiced with human and all mammalian tau isoforms (even isoforms and species variants heretofore undiscovered. Bovine tau is commercially available as a mixture of isoforms.(Sigma Cat. # T7675). A bovine tau sequence is cataloged in Genbank as accession # M26157. A mouse sequence is cataloged in Genbank as NM_010838 A rat sequence is cataloged in Genbank as NM_017212. Because of the high degree of homology between the tau proteins derived from other mammals it is contemplated that sequence from other species are easily obtainable via the polymerase chain reaction (PCR) via library screening and/or hybridization.

The term " beta -amyloid peptide" refers to a 39-43 amino acid peptide having a molecular weight of about 4.2 kD, which peptide is substantially homologous to the form of the protein described by Glenner, et al. (22) including mutations and post-translational modifications of the normal beta -amyloid peptide. In whatever form, the beta -amyloid peptide is an approximately 39-43 amino acid fragment (differing at the carboxy terminus of the fragment) of a large membrane-spanning glycoprotein, referred to as the beta -amyloid precursor protein (APP). Its 43-amino acid sequence is: (SEQ ID NO: 7)

```
1Asp Ala Glu Phe Arg His Asp Ser Gly Tyr Glu Val His His
15Gln Lys Leu Val Phe Phe Ala Glu Asp Val Gly Ser Asn Lys
29Gly Ala Ile Ile Gly Leu Met Val Gly Gly Val Val Ile Ala
43Thr
```

or a sequence which is homologous thereto. By way of example the corresponding rat and mouse sequences are about 95% homologous to the sequence above, are intended to be encompassed by this definition and can be deduced from Genbank accession number P08592 and AAB41502 respectively. Other homologues are easily deduced from public and private databases or via cloning and sequencing the corresponding APP from the appropriate species.

A beta -amyloid peptide may exist in either soluble or insoluble form. After a concentration-dependent lag period during in vitro incubations, soluble preparations of synthetic beta AP slowly form fibrillar aggregates that resemble natural amyloid and are separable from the aqueous medium by sedimentation. For convenience, in this specification, beta amyloid polypeptide is often referred to as Aβ 1-39, Aβ 1-40, Aβ 1-41, Aβ 1-42, Aβ 1-43 or simply "Aβ"

The term " aggregated beta -amyloid peptide" refers to beta amyloid peptides in an insoluble state.

The term "homologous" is used here to illustrate the degree of identity between the amino acid sequence of a given polypeptide and another amino acid sequence. The amino acid sequence to be compared with the amino acid sequence of the given polypeptide may be deduced from a DNA sequence, e.g. obtained by hybridization as defined above, or may be obtained by conventional amino acid sequencing methods. The degree of homology is preferably determined on the amino acid sequence of a mature polypeptide, It is preferred that the degree of homology is at least 80%, such as at least 90%, preferably at least 95% or even 98% with the amino acid sequence between the amino acid sequences compared.

Homologous amino acid sequences include those amino acid sequences which contain conservative amino acid substitutions. Percent homology can be determined by, for example, the Gap program (Wisconsin Sequence Analysis Package, Version 8 for Unix, Genetics Computer Group, University Research Park, Madison WI), which uses the algorithm of Smith and Waterman *(Adv. Appl. Math.*, **1981**, *2*, 482-489, which is incorporated herein by reference in its entirety) using the default settings.

The term "complex" as used herein in intended to mean a combination of molecules bonded together and is not intended to convey any particular mode of bonding.

The term "tau-beta amyloid complex" or "tau-β amyloid' complex as used herein is intended to mean a complex between a tau protein derived polypeptide and a beta -amyloid peptide.

**Assays of the Invention**

It has long been noted that both senile plaques and neurofibrillary tangles (NFT) which are both fundamental pathological characteristic of Alzheimer's disease (AD), invariably coexist in the AD brain. The main fibrous component of all neurofibrillary lesions is paired helical filament (PHF) which contains predominantly the abnormally phosphorylated tau, while in the brain, tau is a microtubule-associated protein, which promotes microtubule assembly and stabilization. Amyloid plaques are composed of a core of β-amyloid (Aβ) peptide derived from proteolytic processing of the amyloid precursor protein, APP. There is evidence that considerable Aβ 1-42 is produced intracellularly and it is this intracellular accumulation of Aβ fibrils that may be responsible for the pathogenic process. It has been suggested that accumulation of Aβ within the cytoplasm of neurons affected by AD does not require internalization from an external source. Tau is a highly soluble cytoplasmic protein and it undergoes aggregation to form PHF-tau and NFTs. We have discovered that the addition of the aggregated form of Aβ to human recombinant soluble tau results in an instantaneous insolubility of tau protein in a dose-dependent manner. Accordingly, inhibiting the Aβ:tau interaction is a useful therapeutic intervention point in AD. This invention relates to assays and methods for studying interaction between these two molecules associated with Alzheimer's disease. It will be appreciated that assays which measure the propensity for a tau protein derived polypeptide to associate with aggregated beta-amyloid form the basis of a screening tool for agents which are effective in diminishing this interaction.

Agents that inhibit the formation of bound complexes as compared to a control binding reaction lacking agent are thereby identified as tau -Aβ aggregation inhibiting candidate therapeutic agents.

Administration of an efficacious dose of an agent capable of specifically diminishing the interaction between tau and Aβ can be used as a therapeutic or prophylactic method for treating pathological conditions (e.g.Alzheimer's Disease, Progressive Supranuclear Palsy (PSP), Corticobasal Degeneration (CBD), Down's syndrome, Frontotemporal dementia, Parkinsonism linked to chromosome 17 (FTDP), and Pick's disease ) Assays which monitor tau Aβ binding are of value in screening for therapeutic agents for the above mentioned disease states.. Methods of assessing the propensity of a polypeptide like tau or derivatives of tau to associate with another polypeptide like Aβ or the formation of a tau-beta amyloid complex are well known in the art and illustrative examples are discussed.

The assays described below make use of isolated beta-amyloid either in one of it's molecular forms (typically the 39, 40, 41, 42 or 43 amino acid variants). Such polypeptides can be purchased ( For example from Sigma Biochemicals --Fragment 1-38 Cat # A0189, Fragment 1-40 Cat # A1075, Fragment 1-42 Cat # A9810, Fragment 1-43 Cat # A7712 ) Such polypeptides may also be synthetically produced by means well known in the art. Solid state peptide synthesis is well known to those of ordinary skill in the art, and is described generally by Merrifield, 1963, J. Amer. Chem. Soc. 85:2149-2156, Fields and Noble, 1990, Int. J. Pept.. Protein Res. 35:161-214 and Solid Phase Peptide Synthesis: A practical approach" by Atherton and Sheppard (published by IRL press at Oxford University Press, 1989) which are specifically incorporated herein by reference. The peptides and proteins disclosed herein may thus be prepared using these relatively routine techniques given the disclosure of the present invention. Solid state peptide synthesis is well known to those of ordinary skill in the art, as described by the references provided and can be performed manually or by an automated peptide synthesizer such as those sold by ABS.

One method of synthesis is accomplished by solid phase peptide synthesis using the Fmoc strategy using an automated peptide synthesizer. This method involves building an amino acid chain from the -COOH terminus, which is attached to an insoluble polymeric support. The base-labile Fmoc group is used to protect the -amino group of each residue. Residues having potentially reactive side chains are protected with acid-labile groups such as t-butyl. After removal of the Fmoc group during each cycle with piperidine, the next protected amino acid is added using either a coupling reagent or pre-activated amino acid derivative. At the end of the synthesis, the peptide is cleaved from the solid support to yield a peptide acid or amide, depending on the linking agent used, and the side-chain protecting groups are removed by treating the peptide-resin with a mixture of trifluoroacetic acid and various ion scavengers. Methyl t-butyl ether is added to precipitate the peptide out of the cleavage mixture. The crude peptide is dissolved and lyophilized, after which it was purified by high performance liquid chromatography. The purified peptide is lyophilized and stored at - 80° C.

Such polypeptides can also be produced as a consequence of the normal processing of the beta -amyloid precursor protein (APP) and subsequent purification.

The assays described below also make use of isolated tau protein derived polypeptides these polypeptides can be synthetically produced by means well known in the art or they may be isolated in the context of a full length tau isoform or a fragment of a tau isoform by conventional protein purification methods.

In those situations where it is preferable to partially or completely isolate the tau protein derived polypeptide, purification can be accomplished using standard methods well known to the skilled artisan. Such methods include, without limitation, separation by electrophoresis followed by electroelution, various types of chromatography (immunoaffinity, molecular sieve, and/or ion exchange), and/or high pressure liquid chromatography. In some cases, it may be preferable to use more than one of these methods for complete purification.

Purification of the tau protein derived polypeptide can be accomplished using a variety of techniques. If the polypeptide has been synthesized such that it contains a tag such as hexahistidine or other small peptide such as FLAG (Eastman Kodak Co., New Haven, Conn.) or myc (Invitrogen,Carlsbad, Calif.) at either its carboxyl or amino terminus, it may essentially be purified in a one-step process by passing the solution through an affinity column where the column matrix has a high affinity for the tag or for the polypeptide directly (i.e., a monoclonal antibody specifically recognizing tau). For example, polyhistidine binds with great affinity and specificity to nickel, thus an affinity column of nickel (such as the Qiagen Registered TM nickel columns) can be used for purification of tau/polyHis. (See for example, Ausubel et al., eds., Current Protocols in Molecular Biology, Section 10.11.8, John Wiley & Sons, New York [1993]).

Even if the tau protein derived polypeptide is prepared without a label or tag to facilitate purification. The tau protein derived polypeptide of the invention may be purified by immunoaffinity chromatography. To accomplish this, antibodies specific for tau must be prepared by means well known in the art. Antibodies generated against the tau protein derived polypeptide of the invention can be obtained by administering the polypeptides or epitope-bearing fragments, analogues or cells to an animal, preferably a nonhuman, using routine protocols. For preparation of monoclonal antibodies, any technique known in the art that provides antibodies produced by continuous cell line cultures can be used. Examples include various techniques, such as those in Kohler, G. and Milstein, C., Nature 256: 495-497 (1975); Kozbor et al., Immunology Today 4: 72 (1983); Cole et al., pg. 77-96 in Monoclonal Antibodies and Cancer Therapy, Alan R. Liss, Inc. (1985).

Where tau protein derived polypeptide is prepared without a tag attached, and no antibodies are available, other well known procedures for purification can be used. Such procedures include, without limitation, ion exchange chromatography, molecular sieve chromatography, HPLC, native gel electrophoresis in combination with gel elution, and preparative isoelectric focusing ("Isoprime"machine/technique, Hoefer Scientific). In some cases, two or more of these techniques may be combined to achieve increased purity.

It is understood that in some methods of detection of tau-beta amyloid complex it is desireable to label tau protein. Suitable labeling includes, but is not limited to, radiolabeling by incorporation of a radiolabeled amino acid (e. g., $^{14}C$-labeled leucine, $^{3}H$-labeled glycine, $^{35}S$-labeled methionine), radiolabeling by post-translational radioiodination with $^{125}I$ or $^{131}I$ (e.g., Bolton-Hunter reaction and chloramine T), labeling by post-translational phosphorylation with $^{32}P$ or $^{33}P$ (e.g., with tau protein kinase II [TPK II]) fluorescent labeling by incorporation of a fluorescent label (e.g., fluorescein or rhodamine), or labeling by other conventional methods known in the art.

One method of assessing a protein-protein interaction is a scintillation proximity assay (SPA) as described in several references, such as N. D. Cook, et al., Pharmaceutical Manufacturing International, pages 49-53 (1992); K. Takeuchi, Laboratory Practice, September issue (1992); U.S. Pat. No. 4,568,649 (1986); which are incorporated by reference herein.

## EXAMPLES

### Example 1

### Measuring Tau/Aβ Interaction using a SPA

#### Materials

Aβ peptides and human amylin 1-37 peptide were obtained from Bachem and were dissolved and diluted in 50 % DMSO GSK-3 (**TPK I**) was obtained from New England Biolabs. Human recombinant tau 1-383 isoform and recombinant **TPK II** were purified as described in Evans et al. J. Biol. Chem 275 pp. 24977-24983 (2000).

Plasmid DNA from the DE-105,2 clone (Evans et al.) was isolated and used to transform competent BL-21 (DE-3) cells used for expression with 1 mM IPTG for 4 hr in NS-85 media (23). Cells were collected by centrifugation and the cell pellets were stored at -80°C and used for IMAC purification as described elsewhere (23). The tau protein was eluted with buffer containing 300 mM imidazole and protein containing fractions were determined by absorbance at 280 nM. Samples were run on 12% SDS-PAGE and the tau containing samples were pooled and dialyzed in 50 mM Tris, pH 8.0, 1 mM DTT.

*In vitro reconstitution of TPK II (cdk5/p20)*

In vitro reconstitution of TPK II (cdk5/p20) was performed as described in Evans et al. (23). Briefly, the full-length human cdk5 gene was inserted into the baculovirus genome using standard baculovirus expression vector technology. A sample plaque isolate producing the highest level of cdk5 was plaque purified three times and a single virus plaque was used to make stock virus for cdk5 production. For *in vitro* reconstitution of TPK II, human recombinant native cdk5 was partially purified by Q-Sepharose from baculovirus infected insect cells. The truncated version of human p35 activator protein, p20 (G137-R307), was cloned and expressed in *E. coli* as an ubiquitin fusion containing an internal hexa histidine sequence. For *in vitro* reconstitution and purification of human recombinant TPK II (cdk5/p20), the clone DE-93,4 containing the p20 construct, was grown in NS-85 medium (29) until the $A_{600}$ = 0.4 and induced for 3 h with 1 mM IPTG. The crude *E. coli* extract was adjusted to pH 8.0 using 2 M Tris, centrifuged at 12000xg for 1 hr and the supernatant loaded onto a nickel IMAC column equilibrated in 50 mM Tris, pH 8.0. The column was washed overnight with 50 mM Tris, pH 8.0 followed by buffer containing 50 mM imidazole and was then re-equilibrated with 50 mM Tris, pH 8.0. Partially purified cdk5 was diluted 1:2 with 50 mM Tris, pH 8.0 and loaded onto the IMAC. The column was washed with buffer containing 50 mM imidazole and the complex eluted with buffer containing 300 mM imidazole. Fractions containing TPK II (cdk5/p20) by SDS-PAGE were pooled and dialyzed in 20 mM Tris, pH 8.0, 100 mM NaCl, 1 mM DTT. Further purification of the IMAC isolated TPK II was carried out using immobilized streptavidin (Pierce) followed by affinity chromatography on an ATP agarose column (Sigma).

*Phosphorylation of purified tau*

Purified tau (38 μM) was incubated in the presence of 100 nM TPK II for 4 hrs at 37°C containing 40 mM Tris, pH 7.5, 10 mM $MgCl_2$, 1 mM DTT, 0.5 mM ATP and 100 μCi [$^{33}P$] γATP. The unincorporated [$^{33}P$]γATP was removed by BioRad P6 spin columns using the BioRad protocol.

**The Scintillation Proximity assay**

[$^{33}$P] tau was added at various concentrations to 2 μM Aβ 1-42 and 5 mg/ml antimouse PVT SPA beads in a total volume of 100 μl of 40 mM Tris, pH 7.5, 10 mM $MgCl_2$, 1 mM DTT for 30 min at 37°C. The samples were centrifuged at 12,000 x g in a microcentrifuge and counted using a Packard TriCarb liquid scintillation counter. The background CPM was obtained by incubating varying concentrations of [$^{33}$P] tau with the SPA beads in the absence of Aβ 1-42. Because of the insoluble nature of the tau/Aβ complex, the [$^{33}$P] tau centrifuged into a pellet came in close proximity to the centrifuged SPA bead pellet and SPA counts were the result. Few background SPA counts were seen in the absence of Aβ 1-42 suggesting that the counts obtained were the result of an interaction of [$^{33}$P] tau and Aβ 1-42.

SPA counts were plotted versus [$^{33}$P] tau concentration and the data fitted using a ligand binding-1 site equation in The data obtained from the SPA assay were analyzed according to Equation (1), a rectangular hyperbola widely employed for analyzing solid phase-binding data, to determine the half-saturating level of binding (estimated dissociation constant, $K_D$) using the program Prism (GraphPad),

$$B = \frac{B_{max}[L]}{KD+[L]} \qquad (1)$$

where B is the complex concentration (in CPM). $B_{max}$ is the maximum complex concentration and [L] is the molar concentration of tau. The curve is shown in Figure 1. By varying the concentration of [$^{33}$P] tau and keeping the concentration of Aβ constant (2 μM), a relative $K_D$ of 108 ± 25 nM was calculated (Fig. 1). These SPA counts were inhibited in a dose dependent manner when [$^{33}$P] tau and unlabeled tau were added together to SPA beads in the presence of Aβ 1-42 (data not shown). In contrast, soluble tau was unable to compete with preformed Aβ- [$^{33}$P] tau complex, suggesting Aβ promotes tau aggregation. Our results with the above two independent techniques and using purified and well characterized tau and Aβ argue against any confounding interactions of either Aβ or tau with other assay components or proteins. These SPA counts were inhibited in a dose dependent manner when [$^{33}$P] tau and unlabeled tau were added together to SPA beads and Aβ 1-42 (data not shown). In contrast, soluble tau was unable to compete with preformed Aβ- [$^{33}$P] tau complex, suggesting the tau:Aβ interaction promotes tau aggregation.

**SDS Polyacrylamide Gel Electrophoresis**

The presence of a tau protein derived polypeptide/ beta -amyloid peptide aggregate can be detected by analyzing the aggregate pellet and subsequent SDS-polyacrylamide gel electrophoresis or "SDS-PAGE". SDS-PAGE is well known in the art, and is described in Laemmli, U. K., Nature 227:680 (1970), which is hereby incorporated by reference herein.

**Example 2**

**Assessing Tau/Aβ Interaction by SDS-PAGE**

During Aβ:tau interaction, tau undergoes a physical change from soluble tau to aggregated tau. After combining the two and centrifugation, this change can be followed by detecting tau in the pellet and supernatant fractions on a SDS-PAGE.

In order to perform a time course experiment human recombinant tau was incubated with aggregated Aβ 1-42 for various times under defined conditions. When Aβ:tau interaction was studied by SDS-PAGE, 2 μM tau was incubated with the indicated amount of Aβ 1-42 for the indicated time at 37°C in a total volume of 100 μl containing 40 mM Tris, pH 7.5, 10 mM $MgCl_2$, 1 mM DTT. Aβ peptides were added at 10x concentrations to the assay to achieve the final concentration indicated. Samples were centrifuged at 12,000 x g in a micro centrifuge for 5 min. The supernatants were removed, and the pellets washed once with 40 mM Tris, pH 7.5, 10 mM $MgCl_2$, 1 mM DTT and centrifuged again. The pellets were resuspended in 100 μl buffer, electrophoresed on 12% SDS-PAGE next to the corresponding supernatant, and stained with Coomassie Blue R-250. Tau bands were analyzed by densitometry. As shown in Figure 2, Aβ 1-42 -induced tau aggregation is instantaneous (lanes 3 & 4) Under these conditions, at each time point the tau protein was found to be in the pellet fraction along with Aβ 1-42. The percentage of tau protein in the pellet fraction was determined by densitometry of the SDS-PAGE. Dose response studies show that addition of Aβ 1-42 to soluble tau promotes aggregation of tau in a dose-dependent manner and can be followed by SDS-PAGE. The results of a dose-response study are shown in Figure 3.

We recognized that [$^{33}$P] labelled tau would be convenient reagent for following the association with of tau and Aβ 1-42. The effect of Aβ 1-42 on aggregation of unphosphorylated wild type tau was indistinguishable from TPK II phosphorylated tau (Fig. 3).

Since TPK II is a proline-directed kinase and all the proline-directed phosphorylation sites for TPK II are outside the MT-binding repeat region, we believe that Aβ 1-42 exerts its effect on tau by interacting with its MT-binding repeat domains. To test this hypothesis, four repeat (4R) tau (Q244-390A) (comprising residues 244-390 of SEQ ID NO:2 and residues 186-332 of SEQ ID NO:4) lacking the N-terminal and the C-terminal domains and N-terminal tau (M1-238S) (between residues 1-238 of SEQ ID NO:2 and 1-180 of SEQ ID NO:4 lacking the four repeats and the C-terminal domain were prepared to study the effect of Aβ 1-42 on aggregation. Aβ 1-42-induced aggregation of 4R tau (Q244-390A) was comparable to that observed for the wild type tau, while Aβ 1-42-induced aggregation was not observed with tau that lacked the MT-binding repeat domains (Fig. 3). These results suggest that MT-binding repeat domains are critical for Aβ 1-42/tau interaction, while the N-terminal tau domain (1M-238S) is not essential for Aβ 1-42-induced tau aggregation. Similar results were obtained with aggregated form of Aβ 1-40.

In the above experiments Aβ 1-42 was dissolved in DMSO. Thus, it is possible that the observed tau aggregation was due to the presence of DMSO. However, we found that the presence of DMSO had no effect on tau solubility in the absence of Aβ 1-42 under defined conditions. Likewise, the presence of DTT did not effect Aβ 1-42-induced tau aggregation. Since human tau is known to exist in at least six various isoforms and only one recombinant isoform of tau (1-383) was used in the above studies . it was of interest to determine if the observed effect of Aβ was tau isoform (1-383)-specific. Therefore, we also studied various untagged bovine tau isoforms in the presence of Aβ 1-42. The pelleted bovine tau isoforms were monitored by Western blot using 4G5 antibody to tau. These results showed that all various bovine tau isoforms were insoluble in the presence of Aβ 1-42 in a dose-dependent manner (data not shown). Since recombinant tau (1-383) used in this work contains T7 and octa His affinity tags, the results with bovine tau isoforms also confirm that the observed aggregation of human recombinant tau is indeed due to specific interaction between tau and Aβ 1-42.

In yet another embodiment of an SDS-PAGE based assay, tau and Aβ 1-42 were mixed, centrifuged, and the tau protein in the pellet fraction was resuspended and then treated with TPK II. Using equal concentrations of tau protein substrate, Figure 4 shows that compared to soluble tau, there was a marked increase in TPK II-mediated phosphorylation of aggregated tau due to its interaction with Aβ.

**Measuring Aggregation by Turbidity Measurments**

If concentrations of the tau protein derived polypeptide and the beta - amyloid peptide are sufficiently high aggregation results in changes in turbidity which can be followed over time by spectrophotometry at a suitable wavelength. The skilled artisan recognizes that the wavelength chosen is not necessarily critical because light scattering is being measured and not absorbance.

**Example 3**

**Assessing Tau/Aβ Interaction by Absorbance at 350 nm**

Dose-dependent Aβ 1-42-induced tau insolubility was also followed by monitoring an increase in absorbance at 350 nm as a function of Aβ 1-42 concentration. Results are shown in Figure 5. Tau (14 μM) was mixed with varying concentrations of Aβ 1-42 (from 0 to 60 μM) in a total volume of 100 μl containing 50 mM sodium phosphate, pH 7.0. The samples were incubated in a 96 well microplate at 37°C for 30 min and the absorbance read at 350 nm in a Molecular Devices SpectaMax Plus.

**TPK II-mediated tau phosphorylation**

The interaction between tau and Aβ can also be assessed by following an increase in phosphorylation by TPK II and is exemplified below.

**Example 4**

**Assessing Tau/Aβ Interaction by following increase in phosphorylation by TPK II**

We studied the effect of Aβ 1-42 on TPK II-mediated tau phosphorylation by following incorporation of [$^{33}$P] into tau using a filter binding assay. The filter binding assay was done by incubating 2 μM tau with 3.6 nM TPK II for 10 min (or the time indicated) at 37°C in a total volume of 15 μl containing 40 mM Tris, pH 7.5, 10 mM $MgCl_2$, 1 mM DTT, 0.1 mg/ml BSA and 1 μCi [$^{33}$P]γATP. Aβ peptides were added at 10x concentrations to the assay to achieve the final concentration indicated. After incubation, 10 μl was removed and spotted onto phosphocellulose paper. The papers were washed 3X with 1% phosphoric acid, once with acetone, dried and counted in a Packard Tricarb liquid scintillation counter. Figure 6 shows that under defined conditions, Aβ 1-42 stimulates TPK II-mediated tau phosphorylation by about 8 tol0-fold. There was only about a 2-3 fold stimulation of tau phosphorylation by non-aggregated form of Aβ 1-40 or Aβ 25-35. Figure 7 shows a time course study demonstrating that in the presence of Aβ 1-42 there is about an 8-fold increase in the initial rate of tau phosphorylation by TPK II.

**Gel-Shift Assays**

Aggregation and phosphorylation can be accessed by a gel shift assay (an assay in which complex formation is accessed by a decrease in electrophoretic migation of complexed vs. uncomplexed Aβ. An illustrative example is shown below.

**Example 5**

**Assessing Tau/Aβ Interaction by Gel-Shift**

In another embodiment, increase in TPK II phosphorylation was followed by a gel-shift assay. Gel-shift assays were done by incubating 3 μM tau with 50 nM TPK II and/or 167 units/ml TPK I for the indicated time at 37°C in a total volume of 20 μl containing 40 mM Tris, pH 7.5, 10 mM $MgCl_2$, 1 mM DTT, and 1 mM ATP. Aβ peptides were added at 10x concentrations to the assay to achieve the final concentration indicated. Following incubation the samples were electrophoresed on 12 % SDS-PAGE and stained with Coomassie Blue R-250. As shown in Figure 8, a significant increase in gel shift was observed in the presence of Aβ 1-42 (lane 5). The control peptides, non-aggregated Aβ 1-40 or 25-35 peptide, had no effect on tau in the gel shift assay. These results were confirmed with aggregated Aβ 1-42 purchased from two other commercial sources. The observed increase in tau phosphorylation in the presence of Aβ 1-42 was shown to be both dose (Fig. 9) and time-dependent (Fig. 10), consistent with that shown above in the filter binding assays and SDS-PAGE assays.

**Techniques Utilizing Antibodies to Tau Derived Polypeptides**

Antibodies to native tau or a phosphorylated epitope of phosphorylated tau as can be helpful in determining the propensity of a tau derived polypeptide to associate with Aβ The interaction can the be determined by western blot or by ELISA. The techniques for performing Western blots are well known in the art and to those of skill in the art. The detection of the interaction in a sample may also be carried out by using some of the well known ELISA principles, e. g. direct, catching, competitive and double enzyme linked immunosorbent assay.

**Example 6**

**Assessing Tau/Aβ Interaction by Western Blot**

In yet another embodiment, Western blots can be used to follow Aβ:tau interaction by monitoring phosphorylation of an AD-related phosphoepitope (Thr-231) on human recombinant tau. The Western blots were done by using the same phosphorylation conditions as the gel-shift assay. After incubation for 4 hours, approximately 300 ng tau was electrophoresed on 12 % SDS-PAGE, transferred to nitrocellulose and blocked with 4 % BSA. AT180 antibody was used at a 1:5000 dilution followed by goat anti mouse IgG-peroxidase used at a 1:10000 dilution as the secondary antibody. The blots were developed using the ECL protocol. Phosphorylation of an AD-related epitope (P-Thr-231) in tau can be detected by the AT-180 monoclonal antibody, which almost exclusively recognizes paired helical filaments (PHF's) from AD brain (Goedert 1994). Figure 11 shows that Aβ 1-42 stimulates phosphorylation of Thr-231 by TPK II. Furthermore, studies with a T231A tau mutant confirmed that stimulation of phosphorylation at Thr-231 was indeed due to an interaction between Aβ and wild type tau.

**Example 7**

**Tau/Aβ Interaction by ELISA**

A number of ELISA formats can be used to study tau:Aβ interaction. In one embodiment, Aβ is coated onto microtiter plates at 60 μg/ml dissolved in 50 % DMSO in 50 mM sodium carbonate buffer, pH 9.6. After washing three times with 0.05 % Tween 20, a 2% solution of milk extract in PBS (137 mM sodium chloride/10 mM psohphate/2.68 mM KCl, pH 7.4) was added as a blocker. The plate is washed three times with PBS and varying concentrations of tau

(1 to 10 μM) in PBS are added. After 1 hr of incubation at 37 degree C, the plate is washed with PBS. The tau:Aβ interaction can followed by using antibodies to full length tau and a commercally available secondary antibody and a standard ELISA kit. In another embodiment, phosphorylated tau can be used and antibodies to phoshorylated tau can be used as a source of primary antibody. In yet another embodiment. A tagged version of tau can be used and antibodies to the tag can be used as a primary antibody. In a further embodiment, tau can be bound to the solid phase as described above for Aβ. In this case, Aβ is captured by tau on the plates and antibodies to Aβ can be used as a source of primary antibody. In another embodiment tau is captured on the plates and antibodies to tau can be used as a primary antibody.

**Fluorescence Polarization**

When a fluorescently labeled molecule is excited with plane polarized light, it emits light that has a degree of polarization that is inversely proportional to its molecular rotation. Large fluorescently labeled molecules remain relatively stationary during the excited state (4 nanoseconds in the case of fluorescein) and the polarization of the light remains relatively constant between excitation and emission. Small fluorescently labeled molecules rotate rapidly during the excited state and the polarization changes significantly between excitation and emission. Therefore, small molecules have low polarization values and large molecules have high polarization values. It is to be expected then that as a fluorescently labelled molecule is incorporated into an aggregate its polarization value would increase.

**Example 8**

The interaction of tau with Aβ 1-42 was studied using fluorescence polarization. Tau was fluorescently labeled using the Alexa 488 protein labeling kit from Molecular Probes. Alexa labeled tau (16 nM) was incubated with varying concentrations of Aβ 1-42 (from 0 to 50 μM) for 30 min at 37°C in 40 mM Tris, pH 7.5, 10 mM $MgCl_2$, 1 mM DTT. The reactions were read on a fluorescence polarization instrument from Panvera and an increase in polarization studied as a function of increasing Aβ 1-42 concentration. In another embodiment Aβ 1-42 can be synthesized to contain a fluorescein label and the increase in polarization studied as a function of increasing tau concentration.

REP07066EP.txt
**Claims**

**1.** A method for identifying agents that are inhibitors of tau-beta amyloid complex formation, comprising:

(a) contacting a tau protein-derived polypeptide and an aggregated beta-amyloid peptide in the presence and absence of a test agent; and
(b) determining and comparing the respective amounts of tau-beta amyloid complex formed in the presence and absence of the test agent, wherein a test agent which decreases the amount of tau-beta amyloid complex formed is an inhibitor.

**2.** The method of claim 1, wherein the polypeptide comprises residues 244-390 of SEQ ID NO: 2.

**3.** The method of claim 1, wherein the polypeptide comprises residues 186-332 of SEQ ID NO: 4.

**4.** The method of claim 1, wherein the polypeptide comprises residues 186-279 of SEQ ID NO: 6.

**5.** The method of any of claims 1 to 4, wherein the peptide comprises residues 1-39 of SEQ ID NO:7.

**6.** The method of claim 5, wherein the peptide comprises residues 1-40 of SEQ ID NO:7.

**7.** The method of claim 5, wherein the peptide comprises residues 1-41 of SEQ ID NO:7.

**8.** The method of claim 5, wherein the peptide comprises residues 1-42 of SEQ ID NO:7.

**9.** The method of claim 5, wherein the peptide comprises residues 1-43 of SEQ ID NO:7.

**10.** The method of any of claims 1 to 9, wherein the determining comprises a scintillation proximity assay.

**11.** The method of any of claims 1 to 9, wherein the determining comprises polyacrylamide gel electrophoresis.

**12.** The method of any of claims 1 to 9, wherein the determining comprises a turbidity measurement.

**13.** The method of any of claims 1 to 9, wherein the determining comprises a gel shift assay.

**14.** The method of any of claims 1 to 9, wherein the determining comprises antibody binding.

**15.** The method of any of claims 1 to 9, wherein the determining comprises ELISA.

**16.** The method of any of claims 1 to 9, wherein the determining comprises Western blotting.

**17.** The method of any of claims 1 to 9, wherein the determining comprises fluorescence polarisation.

**18.** The method of any of claims 1 to 17, wherein the polypeptide is labelled.

**19.** The method of any of claims 1 to 17, wherein the polypeptide comprises a tag.

*Figure 1*

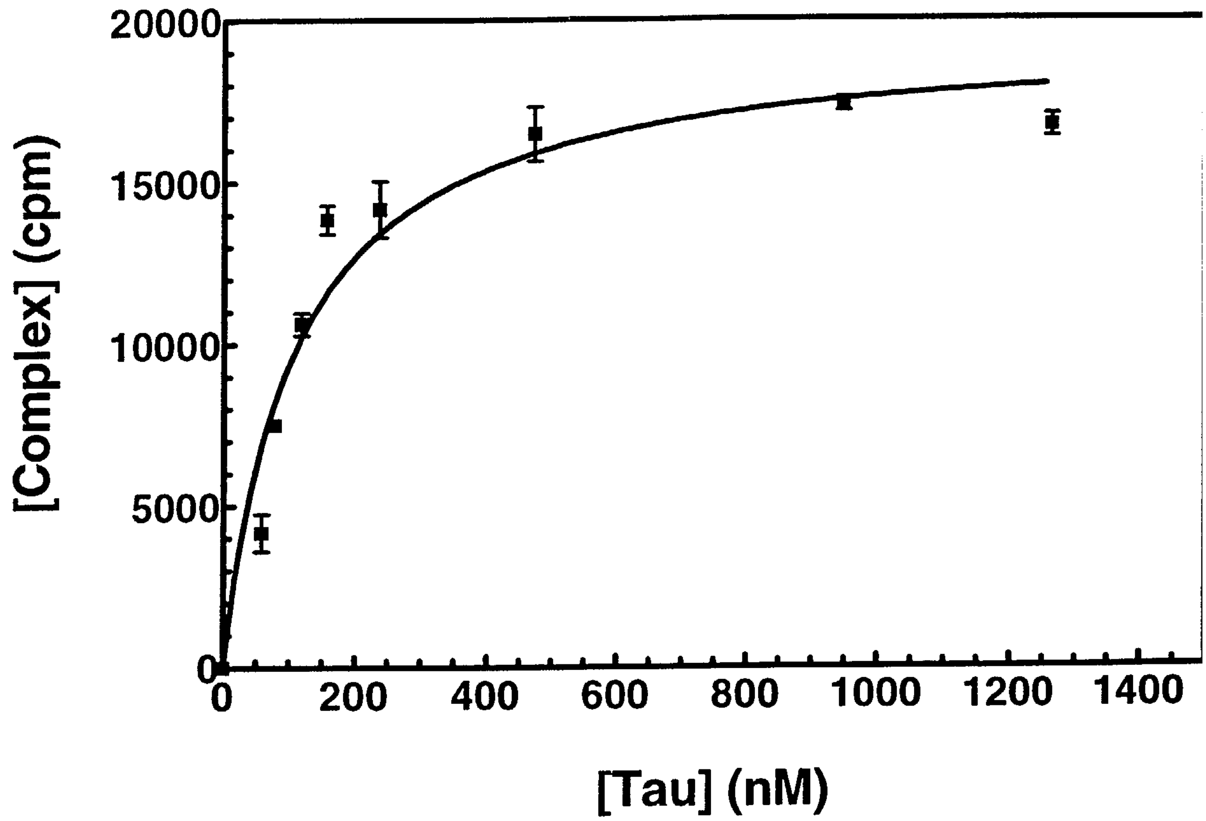
20000
15000
10000
5000
0
[Complex] (cpm)
0
200
400
600
800
1000
1200
1400
[Tau] (nM)

*Figure 2*

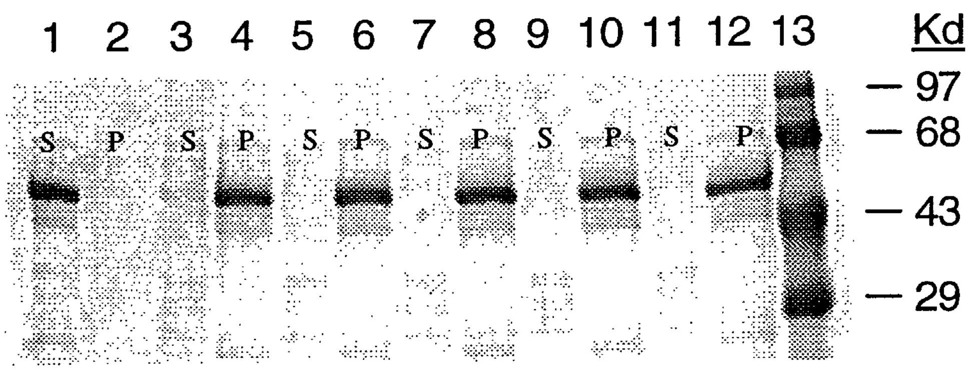

# *Figure 3*

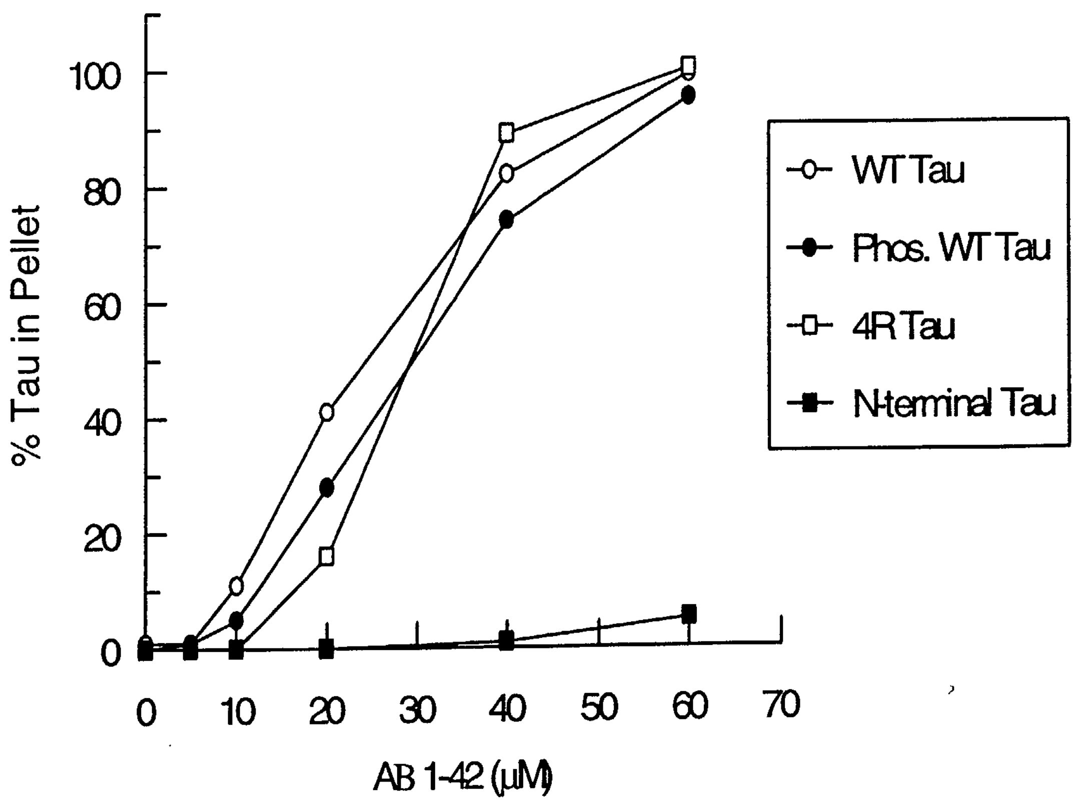
100
80
60
40
20
0
% Tau in Pellet
0
10
20
30
40
50
60
70
AB 1-42 (µM)
WT Tau
Phos. WT Tau
4R Tau
N-terminal Tau

*Figure 4*

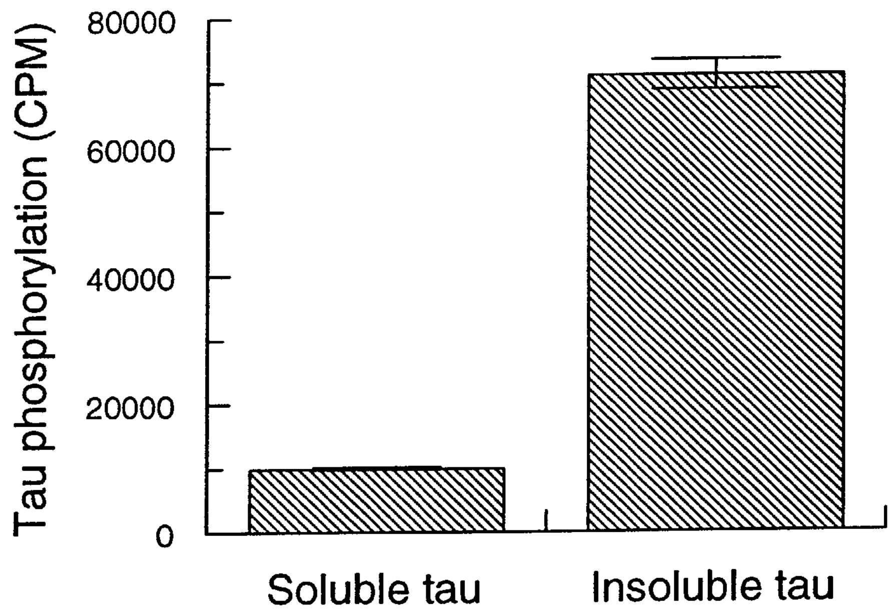


Average of three experiments

*Figure 5*

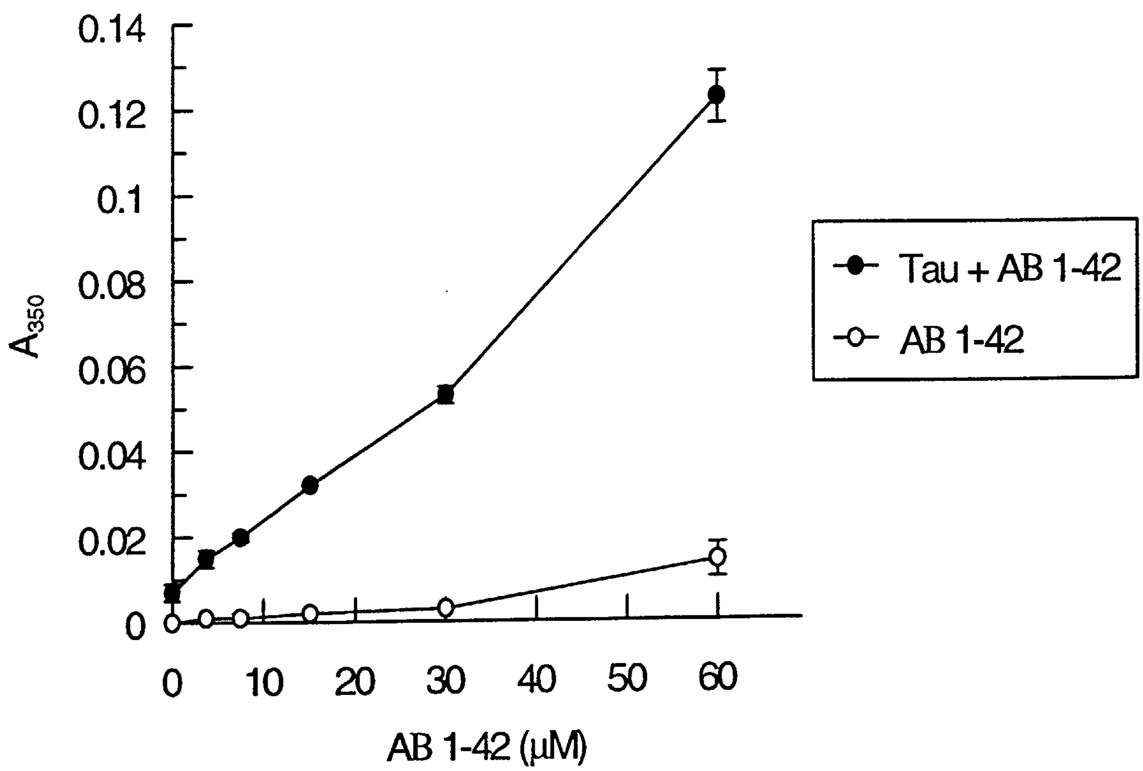
0.14
0.12
0.1
0.08
0.06
0.04
0.02
0
A350
0
10
20
30
40
50
60
AB 1-42 (µM)
Tau + AB 1-42
AB 1-42

*Figure 6*

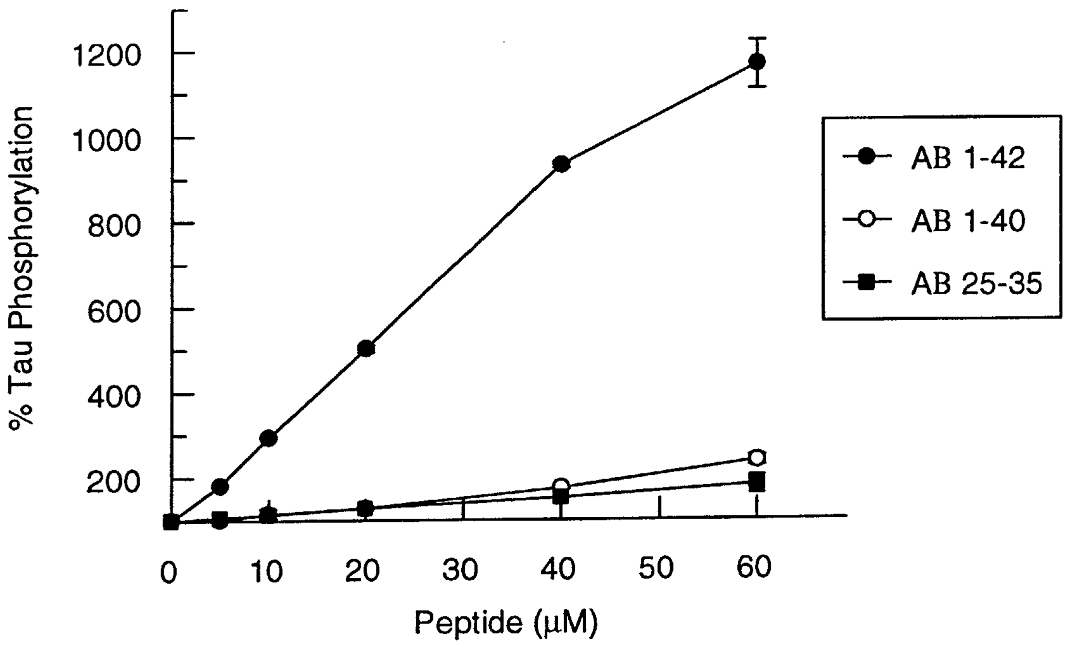
% Tau Phosphorylation
1200
1000
800
600
400
200
0
10
20
30
40
50
60
Peptide (μM)
AB 1-42
AB 1-40
AB 25-35

*Figure 7*

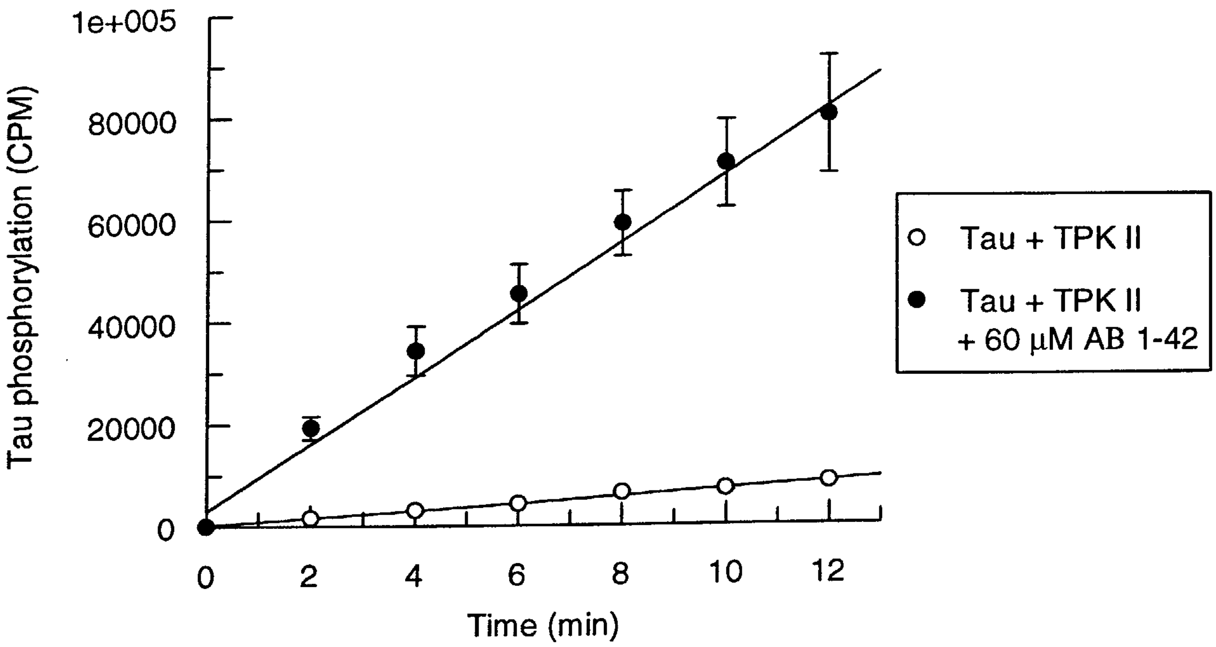
1e+005
80000
60000
40000
20000
0
Tau phosphorylation (CPM)
0
2
4
6
8
10
12
Time (min)
Tau + TPK II
Tau + TPK II
+ 60 μM AB 1-42

*Figure 8*

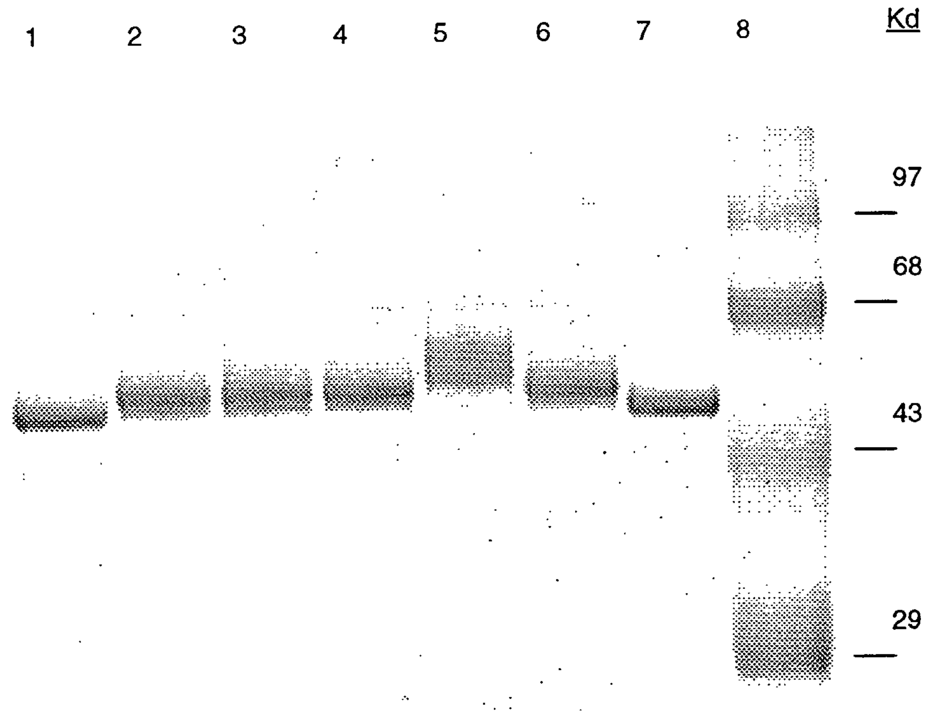

# *Figure 9*

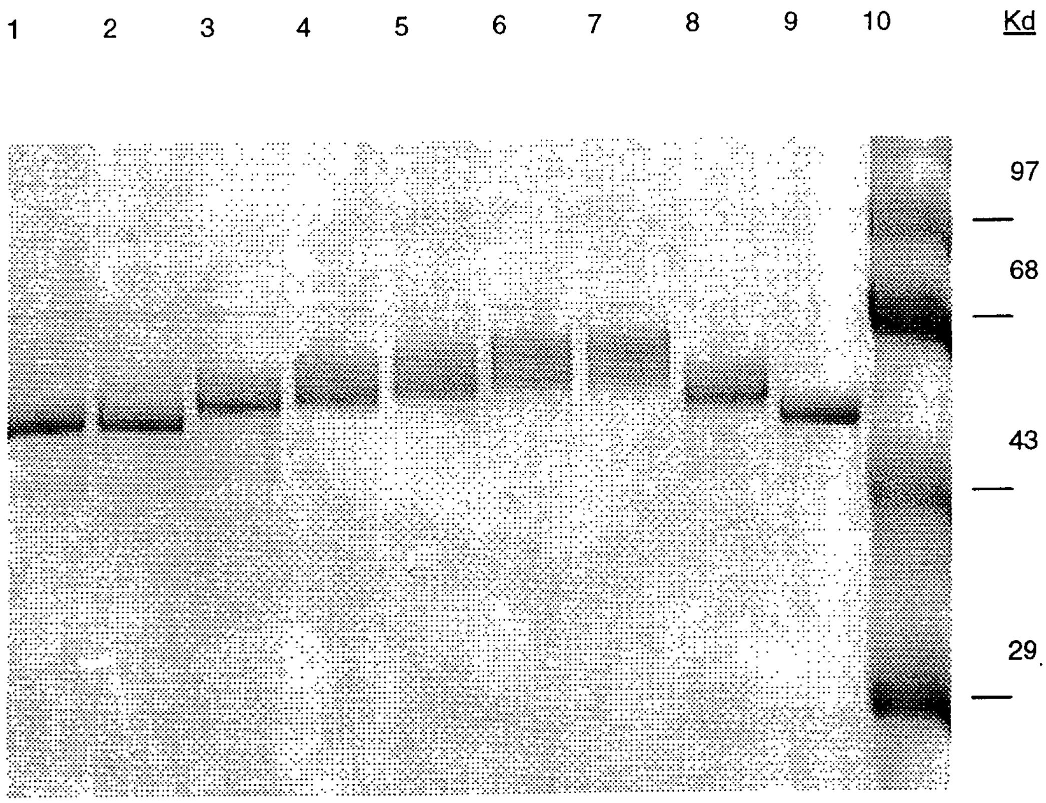

# *Figure 10*

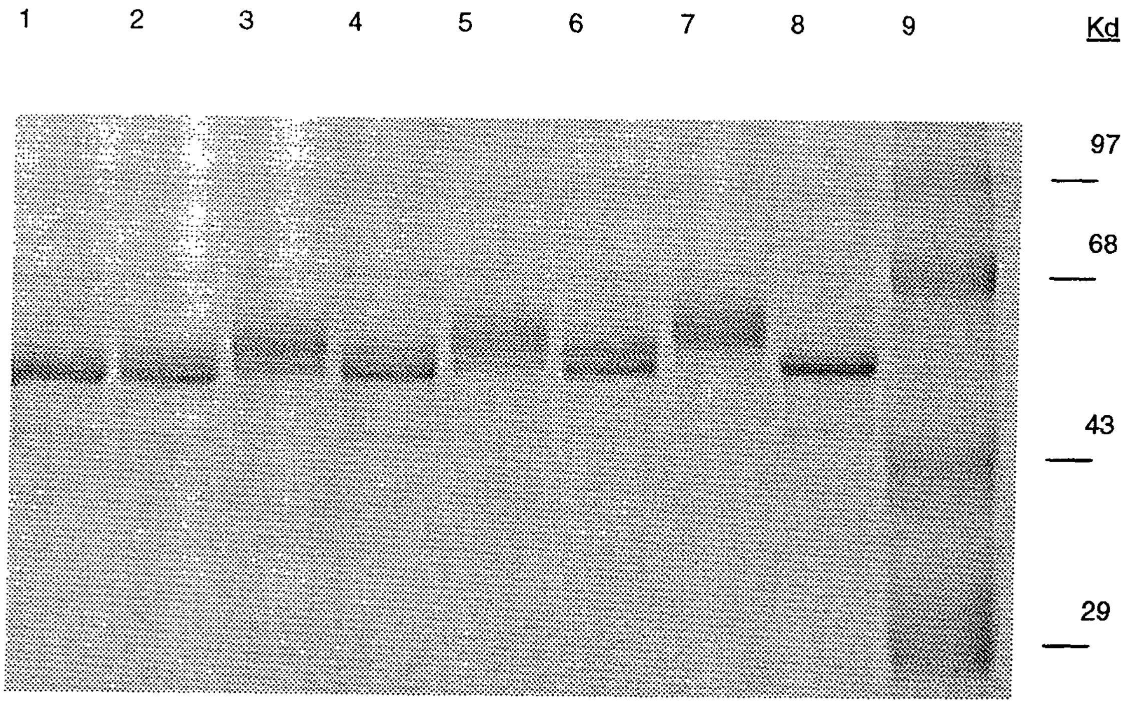

# Figure 11

SEQUENCE LISTING

<110> Pharmacia & Upjohn Company

<120> Assays for Accessing Aß-Tau Aggregation

<130> REP07066EP

<160> 7

<170> PatentIn version 3.0

<210> 1
<211> 2796
<212> DNA
<213> Homo sapiens

<400> 1
```
cctcccctgg ggaggctcgc gttcccgctg ctcgcgcctg ccgcccgccg gcctcaggaa      60

cgcgccctct cgccgcgcgc gccctcgcag tcaccgccac ccaccagctc cggcaccaac     120

agcagcgccg ctgccaccgc ccaccttctg ccgccgccac cacagccacc ttctcctcct     180

ccgctgtcct ctcccgtcct cgcctctgtc gactatcagg tgaactttga accaggatgg     240

ctgagccccg ccaggagttc gaagtgatgg aagatcacgc tgggacgtac gggttggggg     300

acaggaaaga tcaggggggc tacaccatgc accaagacca agagggtgac acggacgctg     360

gcctgaaaga atctcccctg cagaccccca ctgaggacgg atctgaggaa ccgggctctg     420

aaacctctga tgctaagagc actccaacag cggaagatgt gacagcaccc ttagtggatg     480

agggagctcc cggcaagcag gctgccgcgc agccccacac ggagatccca gaaggaacca     540

cagctgaaga agcaggcatt ggagacaccc ccagcctgga agacgaagct gctggtcacg     600

tgacccaagc tcgcatggtc agtaaaagca aagacgggac tggaagcgat gacaaaaaag     660

ccaagggggc tgatggtaaa acgaagatcg ccacaccgcg gggagcagcc cctccaggcc     720

agaagggcca ggccaacgcc accaggattc cagcaaaaac cccgcccgct ccaaagacac     780

cacccagctc tggtgaacct ccaaaatcag gggatcgcag cggctacagc agccccggct     840

ccccaggcac tcccggcagc cgctcccgca ccccgtccct tccaacccca cccacccggg     900

agcccaagaa ggtggcagtg gtccgtactc cacccaagtc gccgtcttcc gccaagagcc     960

gcctgcagac agcccccgtg cccatgccag acctgaagaa tgtcaagtcc aagatcggct    1020

ccactgagaa cctgaagcac cagccgggag gcgggaaggt gcagataatt aataagaagc    1080
```

REP07066EP.txt

```
tggatcttag caacgtccag tccaagtgtg gctcaaagga taatatcaaa cacgtcccgg   1140
gaggcggcag tgtgcaaata gtctacaaac cagttgacct gagcaaggtg acctccaagt   1200
gtggctcatt aggcaacatc catcataaac caggaggtgg ccaggtggaa gtaaaatctg   1260
agaagcttga cttcaaggac agagtccagt cgaagattgg gtccctggac aatatcaccc   1320
acgtccctgg cggaggaaat aaaaagattg aaacccacaa gctgaccttc cgcgagaacg   1380
ccaaagccaa gacagaccac ggggcggaga tcgtgtacaa gtcgccagtg gtgtctgggg   1440
acacgtctcc acggcatctc agcaatgtct cctccaccgg cagcatcgac atggtagact   1500
cgccccagct cgccacgcta gctgacgagg tgtctgcctc cctggccaag cagggtttgt   1560
gatcaggccc ctggggcggt caataattgt ggagaggaga gaatgagaga gtgtggaaaa   1620
aaaaagaata atgacccggc ccccgccctc tgcccccagc tgctcctcgc agttcggtta   1680
attggttaat cacttaacct gcttttgtca ctcggctttg gctcgggact tcaaaatcag   1740
tgatgggagt aagagcaaat ttcatctttc caaattgatg ggtgggctag taataaaata   1800
tttaaaaaaa aacattcaaa aacatggcca catccaacat ttcctcaggc aattcctttt   1860
gattcttttt tcttcccccct ccatgtagaa gagggagaag gagaggctct gaaagctgct   1920
tctgggggat ttcaagggac tgggggtgcc aaccacctct ggccctgttg tgggggttgt   1980
cacagaggca gtggcagcaa caaaggattt gaaactttgg gtgtgttcgt ggagccacag   2040
gcagacgatg tcaaccttgt gtgagtgtga cgggggttgg ggtggggcgg gaggccacgg   2100
gggaggccga ggcaggggct gggcagaggg gaggaggaag cacaagaagt gggagtggga   2160
gaggaagcca cgtgctggag agtagacatc cccctccttg ccgctgggag agccaaggcc   2220
tatgccacct gcagcgtctg agcggccgcc tgtccttggt ggccgggggt gggggcctgc   2280
tgtgggtcag tgtgccaccc tctgcagggc agcctgtggg agaagggaca gcgggttaaa   2340
aagagaaggc aagcctggca ggagggttgg cacttcgatg atgacctcct tagaaagact   2400
gaccttgatg tcttgagagc gctggcctct tcctccctcc ctgcagggta gggcgcctga   2460
gcctaggcgg ttccctctgc tccacagaaa ccctgtttta ttgagttctg aaggttggaa   2520
ctgctgccat gattttggcc actttgcaga cctgggactt tagggctaac cagttctctt   2580
tgtaaggact tgtgcctctt gggagacgtc cacccgtttc caagcctggg ccactggcat   2640
```

REP07066EP.txt

```
ctctggagtg tgtgggggtc tgggaggcag gtcccgagcc ccctgtcctt cccacggcca   2700

ctgcagtcac cccgtctgcg ccgctgtgct gttgtctgcc gtgagagccc aatcactgcc   2760

tatacccctc atcacacgtc acaatgtccc gaattc                             2796


<210>  2
<211>  441
<212>  PRT
<213>  Homo sapiens

<400>  2

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Asp Val Thr Ala Pro Leu Val
65                  70                  75                  80

Asp Glu Gly Ala Pro Gly Lys Gln Ala Ala Ala Gln Pro His Thr Glu
                85                  90                  95

Ile Pro Glu Gly Thr Thr Ala Glu Glu Ala Gly Ile Gly Asp Thr Pro
            100                 105                 110

Ser Leu Glu Asp Glu Ala Ala Gly His Val Thr Gln Ala Arg Met Val
        115                 120                 125

Ser Lys Ser Lys Asp Gly Thr Gly Ser Asp Asp Lys Lys Ala Lys Gly
    130                 135                 140

Ala Asp Gly Lys Thr Lys Ile Ala Thr Pro Arg Gly Ala Ala Pro Pro
145                 150                 155                 160

Gly Gln Lys Gly Gln Ala Asn Ala Thr Arg Ile Pro Ala Lys Thr Pro
                165                 170                 175

Pro Ala Pro Lys Thr Pro Pro Ser Ser Gly Glu Pro Pro Lys Ser Gly
            180                 185                 190

Asp Arg Ser Gly Tyr Ser Ser Pro Gly Ser Pro Gly Thr Pro Gly Ser
        195                 200                 205
```

REP07066EP.txt

```
Arg Ser Arg Thr Pro Ser Leu Pro Thr Pro Pro Thr Arg Glu Pro Lys
    210                 215                 220

Lys Val Ala Val Val Arg Thr Pro Pro Lys Ser Pro Ser Ser Ala Lys
225                 230                 235                 240

Ser Arg Leu Gln Thr Ala Pro Val Pro Met Pro Asp Leu Lys Asn Val
                245                 250                 255

Lys Ser Lys Ile Gly Ser Thr Glu Asn Leu Lys His Gln Pro Gly Gly
            260                 265                 270

Gly Lys Val Gln Ile Ile Asn Lys Lys Leu Asp Leu Ser Asn Val Gln
        275                 280                 285

Ser Lys Cys Gly Ser Lys Asp Asn Ile Lys His Val Pro Gly Gly Gly
    290                 295                 300

Ser Val Gln Ile Val Tyr Lys Pro Val Asp Leu Ser Lys Val Thr Ser
305                 310                 315                 320

Lys Cys Gly Ser Leu Gly Asn Ile His His Lys Pro Gly Gly Gly Gln
                325                 330                 335

Val Glu Val Lys Ser Glu Lys Leu Asp Phe Lys Asp Arg Val Gln Ser
            340                 345                 350

Lys Ile Gly Ser Leu Asp Asn Ile Thr His Val Pro Gly Gly Gly Asn
        355                 360                 365

Lys Lys Ile Glu Thr His Lys Leu Thr Phe Arg Glu Asn Ala Lys Ala
    370                 375                 380

Lys Thr Asp His Gly Ala Glu Ile Val Tyr Lys Ser Pro Val Val Ser
385                 390                 395                 400

Gly Asp Thr Ser Pro Arg His Leu Ser Asn Val Ser Ser Thr Gly Ser
                405                 410                 415

Ile Asp Met Val Asp Ser Pro Gln Leu Ala Thr Leu Ala Asp Glu Val
            420                 425                 430

Ser Ala Ser Leu Ala Lys Gln Gly Leu
        435                 440

<210>  3
<211>  1200
<212>  DNA
<213>  Homo sapiens

<400>  3
tgtcgactat caggtgaact ttgaaccagg atggctgagc cccgccagga gttcgaagtg      60
```

```
atggaagatc acgctgggac gtacgggttg ggggacagga aagatcaggg gggctacacc   120
atgcaccaag accaagaggg tgacacggac gctggcctga aagctgaaga agcaggcatt   180
ggagacaccc ccagcctgga agacgaagct gctggtcacg tgacccaagc tcgcatggtc   240
agtaaaagca aagacgggac tggaagcgat gacaaaaaag ccaagggggc tgatggtaaa   300
acgaagatcg ccacaccgcg gggagcagcc cctccaggcc agaagggcca ggccaacgcc   360
accaggattc cagcaaaaac cccgcccgct ccaaagacac cacccagctc tggtgaacct   420
ccaaaatcag gggatcgcag cggctacagc agccccggct ccccaggcac tcccggcagc   480
cgctcccgca ccccgtccct tccaacccca cccacccggg agcccaagaa ggtggcagtg   540
gtccgtactc cacccaagtc gccgtcttcc gccaagagcc gcctgcagac agcccccgtg   600
cccatgccag acctgaagaa tgtcaagtcc aagatcggct ccactgagaa cctgaagcac   660
cagccgggag gcgggaaggt gcagataatt aataagaagc tggatcttag caacgtccag   720
tccaagtgtg gctcaaagga taatatcaaa cacgtcccgg gaggcggcag tgtgcaaata   780
gtctacaaac cagttgacct gagcaaggtg acctccaagt gtggctcatt aggcaacatc   840
catcataaac caggaggtgg ccaggtggaa gtaaaatctg agaagcttga cttcaaggac   900
agagtccagt cgaagattgg gtccctggac aatatcaccc acgtccctgg cggaggaaat   960
aaaaagattg aaacccacaa gctgaccttc cgcgagaacg ccaaagccaa gacagaccac  1020
ggggcggaga tcgtgtacaa gtcgccagtg gtgtctgggg acacgtctcc acggcatctc  1080
agcaatgtct cctccaccgg cagcatcgac atggtagact cgccccagct cgccacgcta  1140
gctgacgagg tgtctgcctc cctggccaag cagggtttgt gatcaggccc ctggggcggt  1200
```

```
<210>  4
<211>  383
<212>  PRT
<213>  Homo sapiens

<400>  4

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Ala Glu Glu Ala
```

REP07066EP.txt

```
                35                  40                  45

Gly Ile Gly Asp Thr Pro Ser Leu Glu Asp Glu Ala Ala Gly His Val
    50                  55                  60

Thr Gln Ala Arg Met Val Ser Lys Ser Lys Asp Gly Thr Gly Ser Asp
65                  70                  75                  80

Asp Lys Lys Ala Lys Gly Ala Asp Gly Lys Thr Lys Ile Ala Thr Pro
                85                  90                  95

Arg Gly Ala Ala Pro Pro Gly Gln Lys Gly Gln Ala Asn Ala Thr Arg
            100                 105                 110

Ile Pro Ala Lys Thr Pro Pro Ala Pro Lys Thr Pro Pro Ser Ser Gly
        115                 120                 125

Glu Pro Pro Lys Ser Gly Asp Arg Ser Gly Tyr Ser Ser Pro Gly Ser
    130                 135                 140

Pro Gly Thr Pro Gly Ser Arg Ser Arg Thr Pro Ser Leu Pro Thr Pro
145                 150                 155                 160

Pro Thr Arg Glu Pro Lys Lys Val Ala Val Val Arg Thr Pro Pro Lys
                165                 170                 175

Ser Pro Ser Ser Ala Lys Ser Arg Leu Gln Thr Ala Pro Val Pro Met
            180                 185                 190

Pro Asp Leu Lys Asn Val Lys Ser Lys Ile Gly Ser Thr Glu Asn Leu
        195                 200                 205

Lys His Gln Pro Gly Gly Gly Lys Val Gln Ile Ile Asn Lys Lys Leu
    210                 215                 220

Asp Leu Ser Asn Val Gln Ser Lys Cys Gly Ser Lys Asp Asn Ile Lys
225                 230                 235                 240

His Val Pro Gly Gly Gly Ser Val Gln Ile Val Tyr Lys Pro Val Asp
                245                 250                 255

Leu Ser Lys Val Thr Ser Lys Cys Gly Ser Leu Gly Asn Ile His His
            260                 265                 270

Lys Pro Gly Gly Gly Gln Val Glu Val Lys Ser Glu Lys Leu Asp Phe
        275                 280                 285

Lys Asp Arg Val Gln Ser Lys Ile Gly Ser Leu Asp Asn Ile Thr His
    290                 295                 300

Val Pro Gly Gly Gly Asn Lys Lys Ile Glu Thr His Lys Leu Thr Phe
305                 310                 315                 320
```

Arg Glu Asn Ala Lys Ala Lys Thr Asp His Gly Ala Glu Ile Val Tyr
325 330 335

Lys Ser Pro Val Val Ser Gly Asp Thr Ser Pro Arg His Leu Ser Asn
340 345 350

Val Ser Ser Thr Gly Ser Ile Asp Met Val Asp Ser Pro Gln Leu Ala
355 360 365

Thr Leu Ala Asp Glu Val Ser Ala Ser Leu Ala Lys Gln Gly Leu
370 375 380

<210> 5
<211> 2529
<212> DNA
<213> Homo sapiens

<400> 5
cctcccctgg ggaggctcgc gttcccgctg ctcgcgcctg ccgcccgccg gcctcaggaa 60

cgcgccctct cgccgcgcgc gccctcgcag tcaccgccac ccaccagctc cggcaccaac 120

agcagcgccg ctgccaccgc ccaccttctg ccgccgccac cacagccacc ttctcctcct 180

ccgctgtcct ctcccgtcct cgcctctgtc gactatcagg tgaactttga accaggatgg 240

ctgagccccg ccaggagttc gaagtgatgg aagatcacgc tgggacgtac gggttggggg 300

acaggaaaga tcaggggggc tacaccatgc accaagacca agagggtgac acggacgctg 360

gcctgaaagc tgaagaagca ggcattggag acacccccag cctggaagac gaagctgctg 420

gtcacgtgac ccaagctcgc atggtcagta aaagcaaaga cgggactgga agcgatgaca 480

aaaaagccaa gggggctgat ggtaaaacga agatcgccac accgcgggga gcagcccctc 540

caggccagaa gggccaggcc aacgccacca ggattccagc aaaaaccccg cccgctccaa 600

agacaccacc cagctctggt gaacctccaa aatcagggga tcgcagcggc tacagcagcc 660

ccggctcccc aggcactccc ggcagccgct cccgcacccc gtcccttcca accccaccca 720

cccgggagcc caagaaggtg gcagtggtcc gtactccacc caagtcgccg tcttccgcca 780

agagccgcct gcagacagcc cccgtgccca tgccagacct gaagaatgtc aagtccaaga 840

tcggctccac tgagaacctg aagcaccagc cgggaggcgg gaaggtgcaa atagtctaca 900

aaccagttga cctgagcaag gtgacctcca agtgtggctc attaggcaac atccatcata 960

aaccaggagg tggccaggtg gaagtaaaat ctgagaagct tgacttcaag gacagagtcc 1020

agtcgaagat tgggtccctg gacaatatca cccacgtccc tggcggagga aataaaaaga 1080

ttgaaaccca caagctgacc ttccgcgaga acgccaaagc caagacagac cacggggcgg 1140
agatcgtgta caagtcgcca gtggtgtctg gggacacgtc tccacggcat ctcagcaatg 1200
tctcctccac cggcagcatc gacatggtag actcgcccca gctcgccacg ctagctgacg 1260
aggtgtctgc ctccctggcc aagcagggtt tgtgatcagg cccctggggc ggtcaataat 1320
tgtggagagg agagaatgag agagtgtgga aaaaaaaaga ataatgaccc ggcccccgcc 1380
ctctgccccc agctgctcct cgcagttcgg ttaattggtt aatcacttaa cctgcttttg 1440
tcactcggct ttggctcggg acttcaaaat cagtgatggg agtaagagca aatttcatct 1500
ttccaaattg atgggtgggc tagtaataaa atatttaaaa aaaaacattc aaaaacatgg 1560
ccacatccaa catttcctca ggcaattcct tttgattctt ttttcttccc cctccatgta 1620
gaagagggag aaggagaggc tctgaaagct gcttctgggg gatttcaagg gactgggggt 1680
gccaaccacc tctggccctg ttgtgggggt tgtcacagag gcagtggcag caacaaagga 1740
tttgaaaact ttggtgtgtt cgtggagcca caggcagacg atgtcaacct tgtgtgagtg 1800
tgacgggggt tggggtgggg cgggaggcca cgggggaggc cgaggcaggg gctgggcaga 1860
ggggaggagg aagcacaaga agtgggagtg ggagaggaag ccacgtgctg gagagtagac 1920
atccccctcc ttgccgctgg gagagccaag gcctatgcca cctgcagcgt ctgagcggcc 1980
gcctgtcctt ggtggccggg ggtgggggcc tgctgtgggt cagtgtgcca ccctctgcag 2040
ggcagcctgt gggagaaggg acagcgggtt aaaaagagaa ggcaagcctg gcaggagggt 2100
tggcacttcg atgatgacct ccttagaaag actgaccttg atgtcttgag agcgctggcc 2160
tcttcctccc tccctgcagg gtagggcgcc tgagcctagg cggttccctc tgctccacag 2220
aaaccctgtt ttattgagtt ctgaaggttg gaactgctgc catgattttg gccactttgc 2280
agacctggga ctttagggct aaccagttct ctttgtaagg acttgtgcct cttgggagac 2340
gtccacccgt ttccaagcct gggccactgg catctctgga gtgtgtgggg gtctgggagg 2400
caggtcccga gcccccctgtc cttcccacgg ccactgcagt caccccgtct gcgccgctgt 2460
gctgttgtct gccgtgagag cccaatcact gcctataccc ctcatcacac gtcacaatgt 2520
cccgaattc 2529

<210> 6

REP07066EP.txt

<211> 352
<212> PRT
<213> Homo sapiens

<400> 6

```
Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Ala Glu Glu Ala
        35                  40                  45

Gly Ile Gly Asp Thr Pro Ser Leu Glu Asp Glu Ala Ala Gly His Val
    50                  55                  60

Thr Gln Ala Arg Met Val Ser Lys Ser Lys Asp Gly Thr Gly Ser Asp
65                  70                  75                  80

Asp Lys Lys Ala Lys Gly Ala Asp Gly Lys Thr Lys Ile Ala Thr Pro
                85                  90                  95

Arg Gly Ala Ala Pro Pro Gly Gln Lys Gly Gln Ala Asn Ala Thr Arg
            100                 105                 110

Ile Pro Ala Lys Thr Pro Pro Ala Pro Lys Thr Pro Pro Ser Ser Gly
        115                 120                 125

Glu Pro Pro Lys Ser Gly Asp Arg Ser Gly Tyr Ser Ser Pro Gly Ser
    130                 135                 140

Pro Gly Thr Pro Gly Ser Arg Ser Arg Thr Pro Ser Leu Pro Thr Pro
145                 150                 155                 160

Pro Thr Arg Glu Pro Lys Lys Val Ala Val Val Arg Thr Pro Pro Lys
                165                 170                 175

Ser Pro Ser Ser Ala Lys Ser Arg Leu Gln Thr Ala Pro Val Pro Met
            180                 185                 190

Pro Asp Leu Lys Asn Val Lys Ser Lys Ile Gly Ser Thr Glu Asn Leu
        195                 200                 205

Lys His Gln Pro Gly Gly Gly Lys Val Gln Ile Val Tyr Lys Pro Val
    210                 215                 220

Asp Leu Ser Lys Val Thr Ser Lys Cys Gly Ser Leu Gly Asn Ile His
225                 230                 235                 240

His Lys Pro Gly Gly Gly Gln Val Glu Val Lys Ser Glu Lys Leu Asp
                245                 250                 255
```

REP07066EP.txt

```
Phe Lys Asp Arg Val Gln Ser Lys Ile Gly Ser Leu Asp Asn Ile Thr
            260                 265                 270

His Val Pro Gly Gly Gly Asn Lys Lys Ile Glu Thr His Lys Leu Thr
        275                 280                 285

Phe Arg Glu Asn Ala Lys Ala Lys Thr Asp His Gly Ala Glu Ile Val
    290                 295                 300

Tyr Lys Ser Pro Val Val Ser Gly Asp Thr Ser Pro Arg His Leu Ser
305                 310                 315                 320

Asn Val Ser Ser Thr Gly Ser Ile Asp Met Val Asp Ser Pro Gln Leu
                325                 330                 335

Ala Thr Leu Ala Asp Glu Val Ser Ala Ser Leu Ala Lys Gln Gly Leu
            340                 345                 350

<210>  7
<211>  43
<212>  PRT
<213>  Homo sapiens

<400>  7

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val Ile Ala Thr
        35                  40
```